# EUROPÄISCHE PATENTANMELDUNG

(11) **EP 0 691 125 A1**
(43) Veröffentlichungstag der Anmeldung: **10.01.1996**
(21) Anmeldenummer: 95109371.5
(22) Anmeldetag: 16.06.1995
(51) Int. Cl.: A61K 7/32

(54) **Desodorierende Wirkstoffkombinationen auf der Basis von Alpha-Omega-Alkandicarbonsäuren und Monocarbonsäureestern von Oligoglycerinnen**

(30) Priorität: 04.07.1994 DE 4423410
(71) Anmelder: Beiersdorf Aktiengesellschaft, D-20245 Hamburg (DE)
(72) Erfinder: Wolf, Florian, Dr., D-20251 Hamburg (DE); Klier, Manfred, Dr., D-21521 Aumühle (DE); Traupe, Bernd, D-22457 Hamburg (DE)

(57) **Zusammenfassung**

Kosmetische Desodorantien, enthaltend Gemische aus
I) einer oder mehreren α,Ω-Alkandicarbonsäuren und
II) einem oder mehreren Monocarbonsäureestern des Di- und/oder Triglycerins.

## Beschreibung

Desodorierende Wirkstoffkombinationen auf der Basis von α,Ω-Alkandicarbonsäuren und Monocarbonsäureestern von Oligoglycerinen
Die vorliegende Erfindung betrifft kosmetische Wirkstoffkombinationen, insbesondere Wirkstoffkombinationen als wirksames Prinzip in kosmetischen Desodorantien.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschenderweise gefunden, und darin liegt die Lösung all dieser Aufgaben, daß kosmetische Desodorantien, enthaltend Gemische aus
I) einer oder mehreren α,Ω-Alkandicarbonsäuren und
II) einem oder mehreren Monocarbonsäureestern des Di- und/oder Triglycerins
den Nachteilen des Standes der Technik abhelfen.

Zwar ist bekannt, daß Fettsäureester des Glycerins (also des Monoglycerins) gewisse antimikrobielle Wirkung zeitigen. Bekannt ist ferner, Monoglycerinfettsäureester in desodorierenden Kosmetika einzusetzen, insbesondere das Glycerinmonolaurat. Dennoch bleibt die Wirkung dieser Monoglycerinester weit hinter der der erfindungsgemäßen Monocarbonsäureester zurück.

Ferner beschreibt die Europäische Patentanmeldungsschrift EP-0 036 134 Desodorierende Zusammensetzungen, gekennzeichnet durch einen Gehalt an Derivaten mittel- bis längerkettiger Alkansäuren, welche auch die α,Ω-Alkandicarbonsäuren der allgemeinen Formel 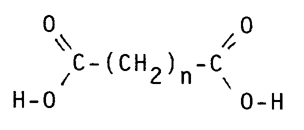
umfassen,
mit n = 4 bis 10, ein Hinweis auf die hiermit vorgelegte Lehre findet sich in dieser Schrift jedoch nicht.

Ferner beschreibt die Deutsche Offenlegungsschrift DE-OS 27 03 642 desodorierende Mittel für die Körperhygiene, welche unter anderem gewisse α,Ω-Alkandicarbonsäuren umfaßt, ein Hinweis auf die hiermit vorgelegte Lehre findet sich jedoch auch in dieser Schrift nicht.

Insbesondere konnte der Stand der Technik keinen Hinweis darauf erkennen lassen, daß sich die erfindungsgemäßen Gemische durch überadditive, also synergistische Wirkung auszeichnen.

Erfindungsgemäß liegen die Di- bzw. Triglycerineinheiten der erfindungsgemäßen Monocarbonsäureester als lineare, unverzweigte Moleküle, also über die jeweiligen OH-Gruppen in 1- bzw. 3-Stellung veretherte "Monoglycerinmoleküle" vor. 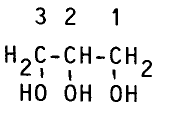
(Glycerin = "Monoglycerin")
Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäureester des Diglycerins sind vorzugsweise Monocarbonsäuremonoester und bevorzugt durch folgende Struktur gekennzeichnet (Substitutionspositionen angegeben): 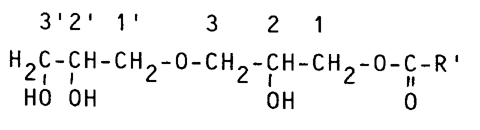
wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind vorzugsweise Monocarbonsäuremonoester und bevorzugt durch folgende Struktur gekennzeichnet (Substitutionspositionen angegeben): 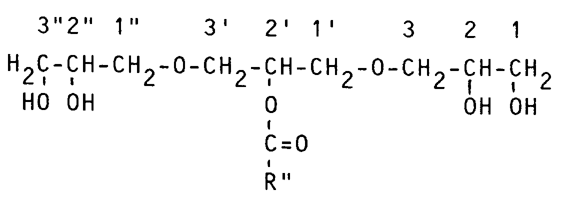
wobei R'' einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die
Hexansäure (Capronsäure) (R' bzw. R'' = -C₅H₁₁),
Heptansäure (Önanthsäure) (R' bzw. R'' = -C₆H₁₃),
Octansäure (Caprylsäure) (R' bzw. R'' = -C₇H₁₅),
Nonansäure (Pelargonsäure) (R' bzw. R'' = -C₈H₁₇),
Decansäure (Caprinsäure) (R' bzw. R'' = -C₉H₁₉),
Undecansäure (R' bzw. R'' = -C₁₀H₂₁),
10-Undecensäure (Undecylensäure) (R' bzw. R'' = -C₁₀H₁₉),
Dodecansäure (Laurinsäure) (R' bzw. R'' = -C₁₁H₂₃),
Tridecansäure (R' bzw. R'' = -C₁₂H₂₅),
Tetradecansäure (Myristinsäure) (R' bzw. R'' = -C₁₃H₂₇),
Pentadecansäure (R' bzw. R'' = -C₁₄H₂₉),
Hexadecansäure (Palmitinsäure) (R' bzw. R'' = -C₁₅H₃₁),
Heptadecansäure (Margarinsäure) (R' bzw. R'' = -C₁₆H₃₃),
Octadecansäure (Stearinsäure) (R' bzw. R'' = -C₁₇H₃₅).

Besonders günstig werden R' und R'' gewählt aus der Gruppe der unverzweigten Alkylreste mit ungeraden C-Zahlen, insbesondere mit 9, 11 und 13 C-Atomen.

Im allgemeinen sind die Ester des Diglycerins denen des Triglycerins bevorzugt.

Ganz besonders günstig sind
Diglycerinmonocaprinat (DMC) R' = 9
Triglycerinmonolaurat (TML) R'' = 11
Diglycerinmonolaurat (DML) R' = 11
Triglycerinmonomyristat (TMM) R'' = 13.

Als bevorzugter erfindungsgemäßer Monocarbonsäureester hat sich das Diglycerinmonocaprinat (DMC) erwiesen.

Die erfindungsgemäßen Monofettsäureester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2''-Position Asymmetriezentren. Erfindungsgemäß aktiv und gleichermaßen von Vorteil sind die 2S2'S2''S-, die 2R2'S2''S-, die 2S2'R2''S-, die 2R2'R2''S-, die 2S2'S2''R, die 2R2'S2''R-, die 2S2'R2''R- und die 2R2'R2''R-Konfiguration.

Es hat sich als günstig herausgestellt, racemische Gemische der Stereoisomeren zu verwenden.

Nach einer vorteilhaften Ausführungsform der vorliegenden Erfindung werden Gemische eines oder mehrerer Monocarbonsäureester des Diglycerins mit einem oder mehreren Monocarbonsäureestern des Triglycerins verwendet.

Nach einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit anderen in Kosmetika üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf das 100 Gew.-Teile der Gesamtmenge, welche sich aus dem Monocarbonsäureester bzw. den Monocarbonsäureestern des Diglycerins und/oder des Triglycerins und diesen Ersatzwirkstoffen und/oder Verschnittstoffen zusammensetzt.

Nach einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit anderen desodorierend oder das Wachstum von Bakterien hemmend oder Bakterien abtötend wirkenden Stoffen eingesetzt.

Nach noch einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit Monocarbonsäureestern des Glycerins (also des "Monoglycerins") eingesetzt. Dabei übernehmen diese Monocarbonsäureester des Glycerins die Rolle der Verschnittstoffe und/oder Ersatzwirkstoffe und werden vorzugsweise in einer Konzentration bis zu 50 Gew.-Teilen, bevorzugt bis zu 35 Gew.-Teilen eingesetzt, bezogen auf 100 Gew.-Teile der Gesamtmenge, welche sich aus dem Monocarbonsäureester bzw. den Monocarbonsäureestern des Diglycerins und/oder des Triglycerins und diesen Monocarbonsäureestern des Glycerins zusammensetzt.

Solche Monocarbonsäureester des Glycerins sind günstig gekennzeichnet durch eine Struktur wie folgt: 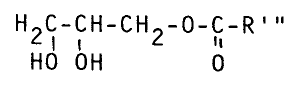
wobei R''' einen Kohlenwasserstoffrest, vorteilhaft einen verzweigten oder unverzweigten Alkyl- oder Alkenylrest von 5 bis 17 C-Atomen darstellt.

Die desodorierende Eigenschaft der erfindungsgemäßen Ester beruht in erster Linie auf deren selektiver Wirksamkeit gegen grampositive, insbesondere coryneforme Bakterien. Diese werden als die für die Zersetzung des apokrinen Schweißes hauptsächlich verantwortlichen Keime angesehen.

Ferner sind die erfindungsgemäßen Ester gut wirksam gegen Staphylococcen.

Da die erfindungsgemäßen Ester zugleich völlig unschädlich für den Menschen und andere Warmblüter sind, sind sie in idealer Weise für die Verwendung in kosmetischen Desodorantien geeignet.

Die erfindungsgemäßen kosmetischen Desodorantien sind besonders vorteilhaft dadurch gekennzeichnet, daß der oder die Monocarbonsäureester des Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Erfindungsgemäß werden die α,Ω-Alkandicarbonsäuren bevorzugt gewählt aus der Gruppe der Substanzen, die von der generischen Formel 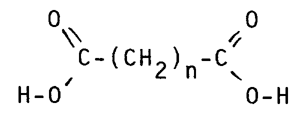
beschrieben werden,
wobei n Zahlen von 1 bis 8 annehmen kann.
- n = 1 :: Malonsäure
- n = 2 :: Bernsteinsäure
- n = 3 :: Glutarsäure
- n = 4 :: Adipinsäure
- n = 5 :: Pimelinsäure
- n = 6 :: Korksäure
- n = 7 :: Azelainsäure
- n = 8 :: Sebacinsäure
Erfindungsgemäß vorteilhaft ist also die Verwendung
I) einer oder mehrerer α,Ω-Alkandicarbonsäuren und
II) eines oder mehrerer Monocarbonsäureester des Di- und/oder Triglycerins
als desodorierend wirkendes Prinzip für kosmetische Desodorantien.

Als besonders vorteilhafte Verkörperung der vorliegenden Erfindung werden kosmetische Desodorantien mit einem Gehalt an
I) Adipinsäure und/oder Azelainsäure und
II) einem oder mehreren Monocarbonsäureestern des Di- und/oder Triglycerins
angesehen.

Als ganz besonders vorteilhafte Verkörperung der vorliegenden Erfindung werden kosmetische Desodorantien mit einem Gehalt an
I) einer oder mehreren α,Ω-Dicarbonsäuren und
II) Diglyerylmonocaprinat
angesehen.

Als bevorzugte Verkörperung der vorliegenden Erfindung werden kosmetische Desodorantien mit einem Gehalt an
I) Adipinsäure und/oder Azelainsäure und
II) Diglyerylmonocaprinat
angesehen.

Erfindungsgemäß ist ferner ein Verfahren zur Bekämpfung des durch mikrobielle Zersetzung apokrinen Schweißes hervorgerufenen menschlichen Körpergeruches, dadurch gekennzeichnet, daß eine wirksame Menge an einem Gemisch aus
I) einer oder mehreren α,Ω-Alkandicarbonsäuren und
II) einem oder mehreren Monocarbonsäureestern des Di- und/oder Triglycerins,
welche gegebenenfalls in einem geeigneten kosmetischen Träger vorliegen können, auf die Haut aufgetragen wird.

Erfindungsgemäß ist schließlich auch die Verwendung eines Gemisches aus
I) einer oder mehreren α,Ω-Alkandicarbonsäuren und
II) einem oder mehreren Monocarbonsäureestern des Di- und/oder Triglycerins,
zur Bekämpfung grampositiver, insbesondere coryneformer Bakterien, beziehungsweise die Verwendung von Monocarbonsäureestern des Di- und/oder Triglycerins zur Verhinderung des Wachstums grampositiver, insbesondere coryneformer Bakterien.

Es ist von Vorteil, den Gehalt an
I) einer oder mehreren α,Ω-Alkandicarbonsäuren und
II) einem oder mehreren Monocarbonsäureestern des Di- und/oder Triglycerins
so zu wählen, daß Verhältnisse von I) und II) wie 5 : 1 bis 1 : 5, insbesondere wie etwa 1 : 1 entstehen.

Die erfindungsgemäßen kosmetischen Desodorantien sind besonders vorteilhaft dadurch gekennzeichnet, daß der oder die Monocarbonsäureester des Di- und/oder Triglycerins in Konzentrationen von 0,05 - 10,00 Gew.-%, bevorzugt 0,1 - 5,0 Gew.-%, vorliegen, jeweils bezogen auf das Gesamtgewicht der Zubereitungen.

Die erfindungsgemäßen kosmetischen Desodorantien sind besonders vorteilhaft dadurch gekennzeichnet, daß die α,Ω-Alkandicarbonsäure oder die α,Ω-Alkandicarbonsäuren in Konzentrationen von 0,01 - 10,00 Gew.-%, bevorzugt 0,05 - 5,0 Gew.-%, vorliegen, jeweils bezogen auf das Gesamtgewicht der Zubereitungen.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der erfindungsgemäßen desodorierenden Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen kosmetischen Desodorantien, welche vorteilhaft als flüssige Zubereitungen mittels einer Roll-on-Vorrichtung auf die gewünschten Hautbereiche aufgetragen werden sollen, und die in den Zubereitungen in geringer Menge, z.B. 2 bis 5 Gewichts.-%, bezogen auf die Gesamtzusammensetzung, verwendet werden können, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetylstearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen desodorierenden kosmetischen Zubereitungen, deren pH-Wert vorzugsweise z.B. durch übliche Puffergemische auf 4,0 bis 7,0 insbesondere 5,0 bis 6,5, eingestellt wird, Parfüm, Farbstoffe, Antioxidantien (z.B. α-Tocopherol und seine Derivate oder Butylhydroxytoluol (BHT = 2,6-Di-tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Der pH-Wert der erfindungsgemäßen kosmetischen Desodorantien wird bevorzugt so eingestellt, daß die erfindungsgemäßen Säurekomponenten im wesentlichen als Säuren, und nicht als Anionen, vorliegen, also bevorzugt im sauren bis neutralen Bereich, insbesondere im pH-Bereich von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen, hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Zusammensetzungen in Pudersprays eingearbeitet werden, so können die Suspensionsgrundlagen dafür vorteilhaft gewählt werden aus der Gruppe

Kieselsäuregele (z.B. solche die unter dem Handelsnamen Aerosil^{R} erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die angegebenen Zahlenwerte beziehen sich stets auf Gew.-%, bezogen auf die Gesamtzusammensetzung, sofern nicht ausdrücklich etwas Anderes vermerkt wird.

| Beispiel 1 - 4 | | | | |
|---|---|---|---|---|
| Pumpspray | 1 | 2 | 3 | 4 |
| Ethanol | 60,00 | 63,00 | 60,00 | 60,00 |
| Propylenglycol PEG-40-hydriertes | 3,00 | 2,50 | 3,00 | 3,00 |
| Rizinusöl | 2,50 | 2,50 | 2,50 | 2,00 |
| Adipinsäure | 0,45 | 0,30 | - | - |
| Azelainsäure | - | - | 0,35 | 0,30 |
| α-Hydroxypalmitinsäure | - | 0,25 | - | - |
| DMC | 0,30 | - | - | - |
| DML | - | 0,30 | - | - |
| TML | - | - | 0,30 | - |
| TMM | - | - | - | 0,30 |
| Parfum | q.s. | q.s. | q.s. | q.s. |
| Wasser, NaOH 10-%ig ad pH 5 | -------------ad 100,00------------- | | | |

| Beispiel 5 - 6 | | |
|---|---|---|
| Roll-on Gel | 5 | 6 |
| Ethanol | 50,00 | 45,00 |
| Hydroxyethylcellulose | 0,50 | 0,50 |
| Steareth-20 ("Brij 78^{R}") | 1,50 | 1,50 |
| Adipinsäure | - | 0,50 |
| Azelainsäure | 0,45 | - |
| DMC | 0,30 | - |
| DML | - | 0,30 |
| Parfum | q.s. | q.s. |
| Wasser, NaOH 10-%ig ad pH 5 | -----ad 100,00------ | |

| Beispiel 7 - 8 | | |
|---|---|---|
| Roll-On Emulsion (O/W) | 7 | 8 |
| Steareth-10 ("Brij 76^{R}") | 4,00 | 4,00 |
| Cetylalkohol | 2,00 | 1,50 |
| Mineralöl DAB 9 | 7,00 | 7,00 |
| PPG-15 Stearylether | 4,50 | 4,50 |
| Methylparaben | 0,20 | 0,20 |
| Dipropylenglycol | 2,50 | 2,50 |
| Adipinsäure | 0,80 | - |
| Bernsteinsäure | - | 1,10 |
| DMC | 0,30 | - |
| DML | - | 0,30 |
| Parfum | q.s. | q.s. |
| Wasser, NaOH 10-%ig ad pH 5 | -----ad 100,00------ | |

| Beispiel 9 - 10 | | |
|---|---|---|
| Wachsstift (Wasserfrei) | 9 | 10 |
| Trilaurin | 38,00 | 38,00 |
| Caprylic/Capric Triglyceride ("Miglyol 812^{R}") | 29,50 | 29,50 |
| Glycerylstearat, selbstemulgierend | 8,50 | 8,50 |
| Bienenwachs | 21,00 | 21,00 |
| Adipinsäure | 0,50 | - |
| Azelainsäure | - | 0,60 |
| DMC | 0,30 | - |
| DML | - | 0,30 |
| Parfum | q.s. | q.s. |
| Wasser, NaOH 10-%ig ad pH 5 | -----ad 100,00------ | |

## Patentansprüche

1. Kosmetische Desodorantien, enthaltend Gemische aus
I) einer oder mehreren α,Ω-Alkandicarbonsäuren und
II) einem oder mehreren Monocarbonsäureestern des Di- und/oder Triglycerins.

2. Kosmetische Desodorantien nach Anspruch 1, dadurch gekennzeichnet, daß die α,Ω-Alkandicarbonsäure bzw. -säuren gewählt werden aus der Gruppe der Substanzen, die von der generischen Formel 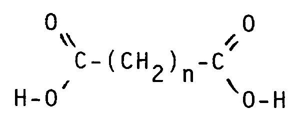 beschrieben werden,
wobei n Zahlen von 1 bis 8 annehmen kann.

3. Kosmetische Desodorantien nach Anspruch 1, dadurch gekennzeichnet, daß als α,Ω-Dicarbonsäure Adipinsäure und/oder Azelainsäure gewählt werden.

4. Kosmetische Desodorantien nach Anspruch 1, dadurch gekennzeichnet, daß die α,Ω-Alkandicarbonsäure oder die α,Ω-Alkandicarbonsäuren in Konzentrationen von 0,01 - 10,00 Gew.-%, bevorzugt 0,05 - 5,0 Gew.-%, vorliegen, jeweils bezogen auf das Gesamtgewicht der Zubereitungen.

5. Kosmetische Desodorantien nach Anspruch 1, dadurch gekennzeichnet, daß der oder die Monocarbonsäureester des Diglycerins durch folgende Struktur gekennzeichnet sind: 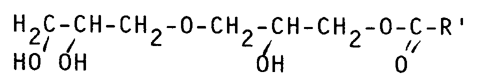 wobei R' einen Kohlenwasserstoffrest, vorteilhaft einen verzweigten oder unverzweigten Alkyl- oder Alkenylrest von 5 bis 17 C-Atomen darstellt.

6. Kosmetische Desodorantien nach Anspruch 1, dadurch gekennzeichnet, daß der oder die Monocarbonsäureester des Triglycerins durch folgende Struktur gekennzeichnet sind: 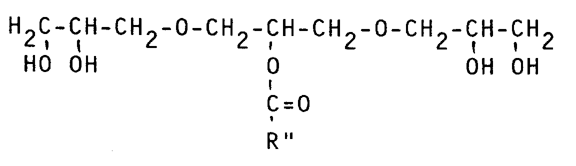 wobei R'' einen Kohlenwasserstoffrest, vorteilhaft einen verzweigten oder unverzweigten Alkyl- oder Alkenylrest Alkylrest von 5 bis 17 C-Atomen darstellt.

7. Kosmetische Desodorantien nach Anspruch 1, dadurch gekennzeichnet, daß der oder die Monocarbonsäureester des Di- bzw. Triglycerins gewählt werden aus der Gruppe Diglycerinmonocaprinat (DMC), Triglycerinmonolaurat (TML), Diglycerinmonolaurat (DML), Triglycerinmonomyristat (TMM).

8. Kosmetische Desodorantien nach Anspruch 1, dadurch gekennzeichnet, daß der Gehalt an
I) einer oder mehreren α,Ω-Alkandicarbonsäuren und
II) einem oder mehreren Monocarbonsäureestern des Di- und/oder Triglycerins
so gewählt wird, daß Verhältnisse von I) und II) wie 5 : 1 bis 1 : 5, insbesondere wie etwa 1 : 1 entstehen.

9. Kosmetische Desodorantien nach Anspruch 1, dadurch gekennzeichnet, daß der oder die Monocarbonsäureester des Di- und/oder Triglycerins in Konzentrationen von 0,05 - 10,00 Gew.-%, bevorzugt 0,1 - 5,0 Gew.-%, vorliegen, jeweils bezogen auf das Gesamtgewicht der Zubereitungen.
